# EUROPEAN PATENT SPECIFICATION

(11) **EP 4 326 163 B1**
(45) Date of publication and mention of the grant of the patent: **16.09.2026**
(21) Application number: 22724873.9
(22) Date of filing: 20.04.2022
(51) Int. Cl.: A61B 10/00

(54) **APPARATUS FOR AND METHOD OF OBTAINING A BIOLOGICAL SAMPLE FROM A SURFACE**
VORRICHTUNG UND VERFAHREN ZUR GEWINNUNG EINER BIOLOGISCHEN PROBE VON EINER OBERFLÄCHE
APPAREIL ET PROCÉDÉ D'OBTENTION D'UN ÉCHANTILLON BIOLOGIQUE À PARTIR D'UNE SURFACE

(30) Priority: 20.04.2021 ZA 202102598
(43) Date of publication of application: 28.02.2024
(73) Proprietor: Cerdak (Pty) Limited, 3867 Mtunzini (ZA)
(72) Inventor: KAMOLZ, Lars-Peter, 8075 Hart bei Graz (AT)
(74) Representative: Maiwald GmbH
(86) International application number: PCT/IB2022/053674
(87) International publication number: WO 2022/224152

(56) References cited:
- EP-A1- 2 523 605
- EP-B1- 2 523 605
- GB-A- 2 506 010
- US-A1- 2004 193 030

## Description

### FIELD OF THE INVENTION

The invention pertains to an apparatus for and a method of obtaining a biological sample from a surface. More particularly, the invention pertains to an apparatus for and a method of obtaining a biological sample from an exposed surface of a human or animal body.

### BACKGROUND TO THE INVENTION

Infection of acute and chronic wounds presents a major impairment to wound healing. Most infected wounds are caused by bacterial colonization, originating either from the normal flora on the skin, or bacteria from other parts of the body or the outside environment.

In order to prescribe the correct treatment for the infected wound, a proper diagnostic assessment has to be performed. The diagnostic assessment is necessary to identify the causative organism (e.g., bacteria) of the infection.

It is known to analyse wound exudate to predict wound healing and to guide wound care. However, currently no biological assay exists to objectively assess wounds to aid in timing of wound closure and guide therapy. Changes in levels of proteases, protease inhibitors, and inflammatory markers have been correlated with wound healing. These findings further support the idea that inflammatory dysregulation and a persistent inflammatory state leads to failure of wound healing in the acute setting. These findings highlight potential targets for the development of a biological assay to individualize management of complex soft-tissue wounds, based on patient physiology and response, that would be applicable to not only military trauma but also civilian trauma. Ultimately, this would result in earlier wound closure, reduction in the number of operating room trips, and reduced health care costs. (Hahm, G., Glaser, JJ., Elster, EA. (2011) Biomarkers to predict wound healing: the future of complex war wound management. Plastic Reconstructive Surgery 127)*.*

A biological marker (biomarker) is a substance used as an indicator of biological state. Advances in genomics, proteomics and molecular pathology have generated many candidate biomarkers with potential clinical value. Research has identified several cellular events and mediators associated with wound healing that can serve as biomarkers. Macrophages, neutrophils, fibroblasts and platelets release cytokines molecules including TNF-α, interleukins (ILs) and growth factors, of which platelet-derived growth factor (PDGF) holds the greatest importance. As a result, various white cells and connective tissue cells release both matrix metalloproteinases (MMPs) and the tissue inhibitors of metalloproteinases (TIMPs). Studies have demonstrated that IL-1, IL-6, and MMPs, levels above normal, and an abnormally high MMP/TIMP ratio are often present in non-healing wounds. Clinical examination of wounds for these mediators could predict which wounds will heal and which will not, suggesting use of these chemicals as biomarkers of wound healing. There is also evidence that the application of growth factors like PDGF will alleviate the recuperating process of chronic, non-healing wounds. Finding a specific biomarker for wound healing status would be a breakthrough in this field and helping treat impaired wound healing. (Patel, S., Maheshwari, A., Chandra, A. (2016); Biomarkers for wound healing and their evaluation - Wound Care (1):46-55).

The so-called swab-rinse technique is most often employed for the diagnostic assessment.

Conventional swabs used in the swab-rinse technique are made from a wooden or plastic shaft with cotton fibres spun to form a bud at one end of the shaft. In performing the swab-rinse technique, the bud is rubbed over the surface of the wound so as to remove bacteria. The bud may, optionally, be moistened in an appropriate wetting agent. Bacteria are then removed from the bud and transferred directly to a solid nutrient medium to develop a laboratory culture. The laboratory culture is then used to identify the causative organism.

The effectiveness of the above-described technique depends, amongst other things, on:
(i) the removal of bacteria from the surface of the wound;
(ii) the release of the bacteria from the bud of the swab; and
(iii) the cultivation of the bacteria.

The above-described technique works only if the swab was performed properly.

It is known that swabbing efficacy is often poor. Studies have reported bacterial recovery rates ranging from 25% to just 0.1% of the original inoculum (*Niskanen and Pohja* 1977; *Roelofsen et al.* 1999; *Moore and Griffith* 2002; *Taku et al.* 2002; *Obee et al.* 2004).

Among the reasons for the poor efficacy of conventional swabbing techniques are inadequate training of a user taking the swab, ineffective absorption and adsorption capability of the bud of the swab and ineffective release of the bacteria from the bud of the swab.

GB2506010 describes microneedle-based devices and methods for the removal of fluid from the body, in particular interstitial fluid, using arrays of hollow or porous microneedles connected to a high-capacity absorbent material such as superabsorbent hydrogel and microporous materials, including porous silica within a pouch or windowed dressing. The system is primarily aimed at therapeutic fluid extraction, such as renal replacement therapy, treatment of oedema and heart failure under negative pressure, rather than non-invasive sampling of wound exudate using a sachet of discrete ceramic particles.

EP2523605 A1 describes a device for transiently contacting at least one capture unit with a body fluid, in which a sampling tip, such as a catheter or needle, carries a capture substrate covered with a biocompatible porous polymer gel layer that retains functionalised nanoparticles and allows biological targets to diffuse in from circulating fluids like blood, cerebrospinal fluid or interstitial fluid. After contact, the gel is dissolved, and the particles recovered, typically magnetically, for analysis.

US 2004/0193030 A1 relates to body-fluid collection and analysis using a storage element formed of porous silicon or similar porous media held beneath a cover or dressing and placed against the skin or another body surface, for example, to collect sweat or other excreted fluids over time. The collected analytes are then analysed, particularly by mass-spectrometric techniques, based on their accumulation in the porous silicon.

### OBJECT OF THE INVENTION

It is an object of the present invention to provide an apparatus for and a method of obtaining a biological sample from an exposed surface of a human or animal body with which the applicant believes the above disadvantages may at least be alleviated or which will provide a useful alternative to known apparatuses and methods.

### SUMMARY OF THE INVENTION

The invention is defined by the independent claims. Preferred features are defined in the dependent claims.

### DETAILED DESCRIPTION OF THE INVENTION

The plurality of separate and porous ceramic particles may be formed by a method including the steps of:
(i) milling alumina (Al₂O₃) into a powder consisting of particles having a diameter of approximately 1 micrometre;
(ii) adding a combustible substance to the powder;
(iii) mixing the powder with water to form a paste or slurry;
(iv) forming of particles from paste or slurry to separate particles having a diameter of between 300 and 3000 micrometres; and
(v) heating the ceramic aggregate to a temperature of 1200°C to form an inert microporous solid ceramic body having pore sizes of between 0.3 and 30 micrometres.

Each of the plurality of separate and porous ceramic particles has a diameter of between 30 and 3000 micrometres, are inert and have a porosity of between 25% and 85%.

The pores of the plurality of separate and porous ceramic particles are cellular in nature and have a diameter of between 0.3 and 30 micrometres. The pores are interconnected to one another by means of blow-holes, which form when the combustible substance escapes from the ceramic aggregate when the latter melts at or close to 1200°C.

A plurality of the separate and porous ceramic particles is encased in a liquid and gas permeable sachet to form an apparatus for obtaining a biological sample from an exposed surface of a human or animal body (e.g., a wound). The sachet is a sterile sachet and the separate and porous ceramic particles are packed loosely therein so as to allow air to pervade therethrough. The sachet is formed from an organic, non-woven material.

The plurality of separate and porous ceramic particles has a relatively large surface area and their micro-pores exhibit a significant capillary suction force.

In use, when the apparatus makes contact with the wound of the human or animal body, the plurality of separate and porous ceramic particles exert a capillary suction force on biological material present on the surface of the wound. The biological material is then absorbed, transported and stored in the micro-pores of the plurality of separate and porous ceramic particles. Since each separate and porous ceramic particle is in contact with surrounding particles of the same kind, biological material on the surface of the wound of the human or animal body migrates continuously between the particles to equalize the hydrostatic potential of all the separate and porous ceramic particles in the sachet. There is no driving force for the biological material to leave the micro-pores of the separate and porous ceramic particles. In this manner, a biological sample is obtained from the wound of the human or animal body.

As discussed above, each of the plurality of separate and porous ceramic particles is made from alumina (Al₂O₃). The surfaces of the plurality of alumina (Al₂O₃) particles are highly charged. The surfaces of the alumina (Al₂O₃) particles are relatively rough and have a wetting angle of 0°. Therefore, the alumina particles are easily wetted. The alumina (Al₂O₃) particles typically have a surface area of 0.5 m² per gram of alumina (Al₂O₃). Each gram of alumina (Al₂O₃) comprises 3.5 x 10²¹ oxygen ions (O²⁻ ) and 2.4 x 10²¹ aluminium ions (Al³⁺), a fair portion of which are exposed to the surface of the particles.

It is known that every cell in a living organism contains an electrical charge defined as the difference between charged atoms on either side of the cell's membrane. A proton colloidal solid, on the other hand, may develop a surface charge due to the ionization of side-chain amino acid groups.

In use, when the plurality of separate and porous alumina particles comes into contact with biological material from the wound, the highly charged alumina surfaces (5.9 x 10²¹ ionic charges per gram) are continuously wetted by biological material. The surfaces of the plurality of separate and porous alumina particles are rough and ideal for adsorbing charged colloidal cells, microorganism, biomarkers, deoxyribonucleic acid (DNA) molecules, proteins, and endotoxins and the like to adhere thereto. Adhesion is caused by ionic and electrostatic interactions, hydrogen bonding and charge-transfer interactions. In this manner, strong adsorption takes place and a biological sample is obtained from the wound of the human or animal body.

The biological material includes wound exudate containing any one or more selected from the group consisting of microorganisms, biomarkers, deoxyribonucleic acid (DNA), proteins, cellular molecules, endotoxins or combinations thereof, macrophages, neutrophils, fibroblasts, platelets, cytokines molecules including TNF-α, interleukins (ILs) and growth factors, including platelet-derived growth factor (PDGF), matrix metalloproteinases (MMPs), tissue inhibitors of metalloproteinases (TIMPs), IL-1, IL-6, and MMPs, proteases, protease inhibitors, and inflammatory markers, and the microorganisms include any one or more selected from the group consisting of bacteria, viruses, fungi and parasites.

The apparatus for obtaining a biological sample from an exposed surface (e.g., a wound) of a human or animal body may include an adhesive dressing. When the apparatus is removed from the wound, the biological material siphoned off from the wound locates in the micro-pores as well as on the surfaces of the plurality of separate and porous ceramic particles. This biological material then forms the biological sample obtained from the wound.

The biological sample obtained from the wound is subjected to sonication to disintegrate a biofilm of microorganisms (e.g., bacteria) present in the biological sample and to form a sonication fluid containing microorganisms. Sonication is typically performed at frequencies of 20 kilohertz. The sonication fluid containing microorganisms is then used to cultivate microorganisms for identifying microorganisms present in the sonication fluid containing microorganisms. These microorganisms are typically bacteria and by cultivating the bacteria the different strains of bacteria present in the sonication fluid can be identified. This enables a healthcare practitioner to prescribe the correct treatment for an infected wound of a human or animal patient.

The sonication fluid can also be used to identify biomarkers which are indicative of the stage of wound healing. This will further inform a healthcare practitioner to prescribe the correct treatment for an infected wound of a human or animal patient.

It was found that the biological sample obtained from the wound could be analysed to predict wound healing and to guide wound care. In particular, analysing the levels of macrophages, neutrophils, fibroblasts, platelets, cytokines molecules including TNF-α, interleukins (ILs) and growth factors, including platelet-derived growth factor (PDGF), matrix metalloproteinases (MMPs), tissue inhibitors of metalloproteinases (TIMPs), IL-1, IL-6, and MMPs, proteases, protease inhibitors, and inflammatory markers, provide useful insight to wound care specialists on the status of the wound healing process and assist in predicting progress and prescribing the appropriate treatment regime.

It will be appreciated by those skilled in the art that the invention is not limited to the precise details as described herein and that many variations are possible without departing from the scope of the invention. The scope of the invention is defined by the appended claims solely.

The description is presented by way of example only in the cause of providing what is believed to be the most useful and readily understood description of the principles and conceptual aspects of the invention. In this regard, no attempt is made to show more detail than is necessary for a fundamental understanding of the invention. The words which have been used herein are words of description and illustration, rather than words of limitation.

### REFERENCES

Hahm, G., Glaser, JJ., Elster, EA. (2011) Biomarkers to predict wound healing: the future of complex war wound management. Plastic Reconstructive Surgery 127*.*
Moore, G. and Griffith, C. (2002) Factors influencing recovery of microorganisms from surfaces by use of traditional hygiene swabbing. Dairy Food Environ Sanit 22, 14- 25.
Niskanen, A. and Pohja, M.S. (1977) Comparative studies on the sampling and investigation of microbial contamination of surfaces by the contact plate and swab methods. J Appl Bacteriol 42, 53- 63.
Roelofsen, E., Van Leeuwen, M., Meijer-Severs, G.J., Wilkinson, M.H.F. and Degener, J.E. (1999) Evaluation of the effects of storage in two different swab fabrics and under three different transport conditions on recovery of aerobic and anaerobic bacteria. J Clin Microbiol 37, 3041- 3043.
Obee, P., Griffith, C., Cooper, R., Bennion, N. and Ramsey, C. (2004) Recovery of methicillin resistant Staphylococcus aureus (MRSA) from wet and dry environmental surfaces. American Society for Microbiology, 104th General Meeting. New Orleans, 23- 27 May, 2004.
Patel, S., Maheshwari, A., Chandra, A. (2016); Biomarkers for wound healing and their evaluation. Wound Care (1):46-55.
Taku, A., Gulati, B.R., Allwood, P.B., Palazzi, K., Hedberg, C.W. and Goyal, S.M. (2002) Concentration and detection of caliciviruses from food contact surfaces. J Food Prot 65, 999- 1004.

## Claims

1. An apparatus for obtaining a biological sample from an exposed surface of a human or animal body, the apparatus including a plurality of separate and porous ceramic particles for absorbing and adsorbing biological material from the exposed surface; and a permeable covering for containing the plurality of separate and porous ceramic particles, wherein the biological material having been absorbed and adsorbed by the plurality of separate and porous ceramic particles forms the biological sample,
wherein the plurality of separate and porous ceramic particles have a diameter of between 300 and 3000 micrometres, and
wherein the plurality of separate and porous ceramic particles have a charged surface caused by ionic and electrostatic interaction, hydrogen bonding and charge-transfer interactions.

2. The apparatus according to claim 1, wherein the plurality of separate and porous ceramic particles are inert and have a porosity of between 25% and 85%.

3. The apparatus according to claim 1, wherein the plurality of separate and porous ceramic particles have pores with a diameter of between 0.3 and 30 micrometres and wherein the pores are cellular in nature and are interconnected with one another by means of blow-holes.

4. The apparatus according to claim 1, wherein the permeable covering is in the form of a sterile permeable and wettable sachet formed from an organic, non-woven material.

5. A method of obtaining a biological sample from an exposed skin surface or external wound of a human or animal body, the method including the steps of providing an apparatus according to any one of claims 1 to 4; and contacting the apparatus with the exposed skin surface or external wound to permit the plurality of separate and porous ceramic particles to absorb and adsorb biological material from the exposed surface, wherein the biological material having been absorbed and adsorbed by the plurality of separate and porous ceramic particles forms the biological sample.

6. The method according to claim 5, wherein the exposed surface is an external wound of a human or animal and the biological material includes wound exudate containing any one or more selected from the group consisting of microorganisms, biomarkers, deoxyribonucleic acid (DNA), proteins, cellular molecules, endotoxins or combinations thereof, macrophages, neutrophils, fibroblasts, platelets, cytokines molecules including TNF-α, interleukins (ILs) and growth factors, including platelet-derived growth factor (PDGF), matrix metalloproteinases (MMPs), tissue inhibitors of metalloproteinases (TIMPs), IL-1, IL-6, and MMPs, proteases, protease inhibitors, and inflammatory markers, and the microorganisms include any one or more selected from the group consisting of bacteria, viruses, fungi and parasites.

7. The method according to claim 5 or 6; further comprising the step of subjecting the apparatus to sonication to form a sonication fluid, the sonication step serving to disintegrate a biofilm of microorganisms present in the absorbed and adsorbed biological material so as to form a sonication fluid containing microorganisms.

8. The method according to claim 7, wherein the sonication is performed at frequencies of 20 kilohertz.

## Patentansprüche

1. Eine Vorrichtung zum Gewinnen einer biologischen Probe von einer freiliegenden Oberfläche eines menschlichen oder tierischen Körpers, wobei die Vorrichtung eine Vielzahl von einzelnen und porösen Keramikpartikeln zum Absorbieren und Adsorbieren von biologischem Material von der freiliegenden Oberfläche sowie eine durchlässige Hülle zum Aufnehmen der Vielzahl von einzelnen und porösen Keramikpartikeln beinhaltet, wobei das von der Vielzahl von einzelnen und porösen Keramikpartikeln absorbierte und adsorbierte biologische Material die biologische Probe bildet,
wobei die Vielzahl einzelner und poröser Keramikpartikel einen Durchmesser zwischen 300 und 3000 Mikrometern aufweist, und
wobei die Vielzahl einzelner und poröser Keramikpartikel eine geladene Oberfläche aufweist, die durch ionische und elektrostatische Wechselwirkungen, Wasserstoffbrückenbindungen und Ladungstransfer-Wechselwirkungen verursacht wird.

2. Die Vorrichtung nach Anspruch 1, wobei die Vielzahl einzelner und poröser Keramikpartikel inert ist und eine Porosität zwischen 25 % und 85 % aufweist.

3. Die Vorrichtung nach Anspruch 1, wobei die Vielzahl einzelner und poröser Keramikpartikel Poren mit einem Durchmesser zwischen 0,3 und 30 Mikrometer aufweist und wobei die Poren zellulärer Natur sind und mittels Durchblaslöchern miteinander verbunden sind.

4. Die Vorrichtung nach Anspruch 1, wobei die durchlässige Hülle die Form eines sterilen, durchlässigen und benetzbaren Beutels aufweist, der aus einem organischen Vliesstoff gebildet ist.

5. Ein Verfahren zur Entnahme einer biologischen Probe von einer freiliegenden Hautoberfläche oder einer äußeren Wunde eines menschlichen oder tierischen Körpers, wobei das Verfahren die folgenden Schritte beinhaltet: Bereitstellen einer Vorrichtung gemäß einem der Ansprüche 1 bis 4; und das In-Kontakt-Bringen der Vorrichtung mit der freiliegenden Hautoberfläche oder der äußeren Wunde, damit die Vielzahl einzelner und poröser Keramikpartikel biologisches Material von der freiliegenden Oberfläche absorbieren und adsorbieren kann, wobei das von der Vielzahl einzelner und poröser Keramikpartikel absorbierte und adsorbierte biologische Material die biologische Probe bildet.

6. Das Verfahren nach Anspruch 5, wobei die freiliegende Oberfläche eine äußere Wunde eines Menschen oder Tieres ist und das biologische Material Wundexsudat beinhaltet, das eines oder mehrere Elemente enthält, ausgewählt aus der Gruppe bestehend aus Mikroorganismen, Biomarkern, Desoxyribonukleinsäure (DNA), Proteinen, zellulären Molekülen, Endotoxinen oder Kombinationen davon, Makrophagen, Neutrophilen, Fibroblasten, Thrombozyten, Zytokinmolekülen, einschließlich TNF-α, Interleukinen (ILs) und Wachstumsfaktoren, einschließlich des von Thrombozyten stammenden Wachstumsfaktors (PDGF), Matrix-Metalloproteinasen (MMPs), Gewebe-Inhibitoren von Metalloproteinasen (TIMPs), IL-1, IL-6 und MMPs, Proteasen, Proteaseinhibitoren und Entzündungsmarkern, wobei die Mikroorganismen einen oder mehrere aus der Gruppe bestehend aus Bakterien, Viren, Pilzen und Parasiten beinhalten.

7. Das Verfahren nach Anspruch 5 oder 6, das ferner den Schritt umfasst, die Vorrichtung einer Ultraschallbehandlung zu unterziehen, um eine Ultraschallflüssigkeit zu bilden, wobei der Ultraschallschritt dazu dient, einen Biofilm aus Mikroorganismen, die in dem absorbierten und adsorbierten biologischen Material vorhanden sind, aufzulösen, um eine Mikroorganismen enthaltende Ultraschallflüssigkeit zu bilden.

8. Das Verfahren nach Anspruch 7, wobei die Ultraschallbehandlung bei Frequenzen von 20 Kilohertz durchgeführt wird.

## Revendications

1. Appareil pour l'obtention d'un échantillon biologique provenant d'une surface exposée d'un corps humain ou animal, l'appareil incluant une pluralité de particules céramiques séparées et poreuses pour l'absorption et l'adsorption de matériel biologique provenant de la surface exposée ; et un revêtement perméable pour contenir la pluralité de particules céramiques séparées et poreuses, dans lequel le matériel biologique ayant été absorbé et adsorbé par la pluralité de particules céramiques séparées et poreuses forme l'échantillon biologique,
dans lequel la pluralité de particules céramiques séparées et poreuses présentent un diamètre d'entre 300 et 3000 micromètres, et
dans lequel la pluralité de particules céramiques séparées et poreuses présentent une surface chargée provoquée par une interaction ionique et électrostatique, une liaison hydrogène et des interactions de transfert de charge.

2. Appareil selon la revendication 1, dans lequel la pluralité de particules céramiques séparées et poreuses sont inertes et présentent une porosité d'entre 25 % et 85 %.

3. Appareil selon la revendication 1, dans lequel la pluralité de particules céramiques séparées et poreuses présentent des pores dotés d'un diamètre d'entre 0,3 et 30 micromètres et dans lequel les pores sont de nature cellulaire et sont reliés les uns aux autres au moyen de trous de soufflage.

4. Appareil selon la revendication 1, dans lequel le revêtement perméable se présente sous la forme d'un sachet stérile perméable et mouillable formé à partir d'une matière organique non tissée.

5. Procédé d'obtention d'un échantillon biologique provenant d'une surface cutanée exposée ou d'une plaie externe d'un corps humain ou animal, le procédé incluant les étapes de la fourniture d'un appareil selon l'une des revendications 1 à 4 ; et de la mise en contact de l'appareil avec la surface cutanée exposée ou la plaie externe pour permettre à la pluralité de particules céramiques séparées et poreuses d'absorber et d'adsorber du matériel biologique provenant de la surface exposée, dans lequel le matériel biologique ayant été absorbé et adsorbé par la pluralité de particules céramiques séparées et poreuses forme l'échantillon biologique.

6. Procédé selon la revendication 5, dans lequel la surface exposée est une plaie externe d'un humain ou d'un animal et le matériel biologique inclut un exsudat de plaie contenant un ou plusieurs éléments sélectionnés parmi le groupe consistant en des micro-organismes, des biomarqueurs, de l'acide désoxyribonucléique (ADN), des protéines, des molécules cellulaires, des endotoxines ou des combinaisons de ceux-ci, des macrophages, des neutrophiles, des fibroblastes, des plaquettes, des molécules de cytokines dont TNF-α, des interleukines (IL) et des facteurs de croissance, dont le facteur de croissance dérivé des plaquettes (PDGF), des métalloprotéinases matricielles (MMP), des inhibiteurs tissulaires des métalloprotéinases (TIMP), d'IL-1, d'IL-6 et des MMP, des protéases, des inhibiteurs de protéase, et des marqueurs inflammatoires, et les micro-organismes incluent un ou plusieurs éléments sélectionnés parmi le groupe consistant en des bactéries, des virus, des champignons et des parasites.

7. Procédé selon la revendication 5 ou 6 ; comprenant en outre l'étape de la soumission de l'appareil à une sonication pour former un fluide de sonication, l'étape de sonication servant à désintégrer un biofilm de microorganismes présent dans le matériel biologique absorbé et adsorbé de façon à former un fluide de sonication contenant des microorganismes.

8. Procédé selon la revendication 7, dans lequel la sonication est réalisée à des fréquences de 20 kilohertz.
